**Europäisches Patentamt**

**European Patent Office**

**Office européen des brevets**

(19)

(11) **EP 1 358 841 A1**

(12) **EUROPÄISCHE PATENTANMELDUNG**

(43) Veröffentlichungstag:
05.11.2003 Patentblatt 2003/45

(51) Int Cl.[7]: **A61B 5/0452**, G06F 17/00

(21) Anmeldenummer: 03000093.9

(22) Anmeldetag: 08.01.2003

(84) Benannte Vertragsstaaten:
**AT BE BG CH CY CZ DE DK EE ES FI FR GB GR HU IE IT LI LU MC NL PT SE SI SK TR**
Benannte Erstreckungsstaaten:
**AL LT LV MK RO**

(30) Priorität: **12.02.2002 DE 10205719**
**13.06.2002 DE 10226374**
**16.10.2002 DE 10248213**

(71) Anmelder: **MEUD Systemtechnik GmbH**
**15344 Strausberg (DE)**

(72) Erfinder:
• **Kühn, Roland, Dipl.-Ing.**
**61279 Grävenwiesbach (DE)**
• **Kusch, Sigurd, Prof. Dr. sc. nat.**
**12526 Berlin (DE)**

(74) Vertreter: **Kruspig, Volkmar, Dipl.-Ing. et al**
**Patentanwälte**
**Meissner, Bolte & Partner**
**Widenmayerstrasse 48**
**80538 München (DE)**

(54) **Auswerteverfahren für Elektrokardiogrammdaten**

(57)    Die Erfindung betrifft ein Auswerteverfahren für Elektrokardiogrammdaten zur Aussage über den funktionellen Gesamtzustand eines Organismus. Bei einem ersten Verfahren werden durch eine Korrelationsanalyse eines zeitlich quasiperiodisch aufeinander folgenden Verlaufsmusters eines Elektrokardiogramms ein die Regelmäßigkeit dieses Verlaufsmusters, insbesondere der R-R-Zeitintervalle, beschreibender, diagnostisch aussagekräftiger numerischer Wert $\alpha$ gewonnen und/oder die Korrelation der R-R-Zeitintervalle anhand der Form einer Punktwolke (600) in einem Korrelationsdiagramm untersucht. Bei einem zweiten Auswerteverfahren werden Parametersätze von Modellfunktionen an den zeitlichen Verlauf der Q- und S-Peaks des EKG iterativ angepasst. Bei einem dritten Auswerteverfahren wird eine Verteilung von Maxima (640) eines Rhythmogramms in einer Zählreihe analysiert.

Fig. 6a

**(Forts. nächste Seite)**

650a    660

rel. Maxima
auf 1 normiert

Zahl der R - R - Abstände

Fig. 8b

**Beschreibung**

Die Erfindung betrifft ein Auswerteverfahren für Elektrokardiogrammdaten nach dem Oberbegriff des Anspruchs 1.

Jeder höhere Organismus besteht aus einer Vielzahl unterschiedlicher, hochgradig spezialisierter Zelltypen, die innerhalb des Organismus symbiotisch zusammenleben und genau festgelegte Funktionen erfüllen. Aus diesen bauen sich Gewebe und Organe auf. Die naturwissenschaftliche Forschung hat das Verständnis über das Zusammenwirken dieser Zelltypen und Zellfamilien mit einer Vielzahl von Ergebnissen beträchtlich erweitert. So ist bekannt, dass jeder einzelne Zelltyp ganz speziellen Lebensrhythmen unterworfen ist, die innerhalb eines Organismus präzise aufeinander abgestimmt sein müssen. Beispielsweise erneuern sich bestimmte Zelltypen innerhalb von Minuten, während andere innerhalb von Stunden, Tagen, Monaten oder Jahren ersetzt werden. Derartige Zyklen betreffen nicht nur das Lebensalter von Zellen, sondern auch deren Lebensphasen, wie beispielsweise deren Wachstum, das Ausbilden funktioneller Eigenschaften, gewisse zelluläre Funktionszyklen und dergleichen weitere biologische Parameter.

Für ein korrektes und im Maßstab eines gesamten Organismus sehr fein aufeinander abgestimmtes Zusammenspiel der verschiedenen Zelltypen kommt dem Milieu sowohl innerhalb des gesamten Organismus, als auch an den Orten der Zellen eine besonders wichtige Bedeutung zu. Dieses Milieu stellt eine durch Stoffwechsel und gegenseitige funktionelle Zusammenarbeit der Zelltypen bestimmte Mikroumwelt für die einzelnen Zellen bereit, wodurch eine Vielzahl von Randbedingungen für die Lebenstätigkeit der Zellen vorab festgelegt wird.

Durch Krankheiten oder äußere Umwelteinflüsse wird diese Mikroumwelt stets mehr oder weniger stark beeinflusst, wobei die oben beschriebenen "Lebenszyklen" einzelner Zellen, Zellverbände oder ganzer Gewebe und Organe aus ihrem präzise aufeinander abgestimmten "Takt" geraten können. Meist sind diese Veränderungen reversibel und werden durch Regelmechanismen des Organismus ausgeglichen. Wirken jedoch negative Umwelteinflüsse zu stark oder über einen längeren Zeitraum auf den Organismus ein oder ist der Organismus durch eine chronische oder schwere Krankheit belastet, können diese Regelmechanismen versagen und das präzise Zusammenspiel der Zelltypen, Zellverbände, Gewebe und Organe wird empfindlich gestört oder kann zusammenbrechen.

Ein Beispiel dafür stellen Geschwulsterkrankungen dar, bei denen geschädigte Zellen eines Zellverbandes, Gewebes oder Organs eine Schädigung durch eine ungünstige Mikroumwelt dadurch auszugleichen versuchen, indem diese sich in abnormer Weise vermehren und ihre eigentlichen Aufgaben innerhalb des Organismus nicht mehr wahrnehmen.

Störungen der Funktionszyklen von Zelltypen lassen sich auch einer Vielzahl von Erkrankungen zugrundelegen. In diesem Zusammenhang sei auf die in den letzten Jahren dramatisch angestiegene Zahl von Herz-Kreislaufoder Gefäßerkrankungen verwiesen.

Es besteht vor dem Hintergrund dieser Erkenntnisse die Aufgabe, zum einen Störungen in den Funktionszyklen von Zelltypen, Zellverbänden, Geweben und/oder Organsystemen rechtzeitig festzustellen, bevor eine dauerhafte Schädigung von Regelmechanismen innerhalb des Organismus eintritt, als auch bekannte periodische oder quasiperiodische Vorgänge innerhalb des Organismus geeignet auszuwerten, sodass einem behandelnden Arzt ein zuverlässiges diagnostisches Mittel zur Verfügung gestellt wird.

Erfindungsgemäß wird die oben genannte Aufgabenstellung mit einem Auswerteverfahren für Elektrokardiogrammdaten jeweils nach den unabhängigen Ansprüchen 1, 5 und 9 gelöst, wobei die abhängigen Unteransprüche zweckmäßige Ausgestaltungen und Weiterbildungen darstellen.

Grundgedanke des Verfahrens nach Anspruch 1 ist es, aus aufgezeichneten Daten eines Elektrokardiogramms (EKG) über eine Korrelationsanalyse eines zeitlich quasiperiodisch aufeinanderfolgenden Verlaufsmusters des EKG einen die Regelmäßigkeit dieses Verlaufsmusters beschreibenden, diagnostisch aussagekräftigen und numerisch eindeutigen Wert zu gewinnen.

Bekanntlich besteht ein aufgezeichnetes Elektrokardiogramm aus einer Abfolge quasiperiodisch vorkommender Ausschläge, die in der medizinischen Praxis als eine Abfolge von P-, Q-, R-, S- und T-Zacken oder Peaks bezeichnet werden. Dabei wiederholen sich diese Strukturen aber nicht streng periodisch, sondern sind selbst unter einer gleichbleibenden Belastungssituation gewissen Schwankungen unterworfen. Der Grundgedanke des erfindungsgemäßen Verfahrens ist es, diese Schwankungen zu analysieren und daraus anormale Muster zu erkennen. Erfindungsgemäß wird dies mittels einer Korrelationsanalyse bewerkstelligt, die eine Beziehung zwischen den zeitlich wiederkehrenden Quasiperioden in den Daten eines EKG herstellt und als Ergebnis einen aussagekräftigen diagnostisch verwertbaren numerischen Wert ausgibt.

Als quasiperiodisches Verlaufsmuster bietet sich für das erfindungsgemäße Verfahren eine Abfolge systolischer R-Peaks innerhalb des EKG an. Diese während der Kontraktionsphasen des Herzmuskels erzeugten Ausschläge zeichnen sich durch eine signifikante und sich besonders leicht von einem Untergrund abhebende Größe ihrer Maxima aus, die ihre Identifikation sehr erleichtert. Sie sind damit eine besonders hervorstechende Kenngröße der Herzaktivität.

Im Einzelnen umfasst die Korrelationsanalyse folgende Schritte:

Als erstes werden Zeitintervalle zwischen den R-Peaks in einem bereits vorliegenden EKG aus bereits aufgezeichneten herkömmlich ermittelten EKG-Daten eingelesen. Anschließend werden Zeitintervalldifferenzen ermittelt. Dies erfolgt durch eine Reihe erster Differenzbildungen und/oder einer Reihe zweiter Differenzbildungen, wobei jeweils äquidistant entfernt indizierte Zeitintervalle voneinander subtrahiert werden. Genauer gesagt, bedeutet dies, dass von einem gegebenen Zeitintervall $t_N$ ein Zeitinvervall $\tau_i$ bei der ersten Differenzbildung subtrahiert wird, während bei der zweiten Differenzbildung ein Zeitintervall $\tau_i$ von einem Zeitintervall $\tau_{N+n}$, welches um eine feste Indizierung n gegenüber dem Zeitintervall $\tau_N$ verschoben ist, abgezogen wird.

Die Wahl von n kann in an sich beliebiger Weise erfolgen, wobei n allerdings fest vorgegeben wird sowie N und/oder i durchlaufende Indizes darstellen. Auf diese Weise werden die sich jeweils um den festen Index n unterscheidenden Zeitintervalle $\tau_N$ und $\tau_{N+n}$ miteinander korreliert.

Ein dritter Schritt der Korrelationsanalyse umfaßt ein Erstellen eines mindestens zweidimensionalen Korrelationsdiagramms, indem die Reihe der ersten Differenzbildungen als Ordinatengröße über den jeweils zugeordneten Differenzbildungen der zweiten Reihe, die als Abszissengröße dienen, aufgetragen werden. Das Ergebnis ist eine mindestens zweidimensionale Punktwolke, die charakteristische Verteilungen aufweist und deren Form bereits visuell gewisse Rückschlüsse auf die Quasiperioden der EKG-Struktur zulässt. Es versteht sich, dass die Gestalt der Punktwolke umso aussagekräftiger ist, je mehr Daten aus einem EKG vorliegen. An sich ist die Wahl des Indexabstandes n und die konkrete Methode, aus der die Reihen der Differenzbildungen hervorgehen, beliebig und unterliegt Zweckmäßigkeitsüberlegungen. In den Beschreibungen der Anwendungsbeispiele wird darauf detaillierter eingegangen.

Ein vierter Schritt besteht in Berechnungen eines ersten Mittelwertes beliebiger Art aus der Reihe der ersten Differenzbildungen und eines zweiten Mittelwertes beliebiger Art aus der Reihe der zweiten Differenzbildungen. Als Mittelwerte können insbesondere arithmetisches oder geometrisches Mittel verwendet werden.

Anschließend werden beide Mittelwerte mittels einer Division zueinander in ein Verhältnis gesetzt und aus diesem Quotient ein diagnostisch aussagekräftiger Wert gewonnen. Die Mittelwertbildung beschreibt einen "Schwerpunkt" der Punktwolke in Richtung ihrer Ordinate, bzw. ihrer Abszisse, während sich aus dem Verhältnis beider errechneter Mittel eine Aussage über die Gleichförmigkeit der Punktwolke aus beiden Differenzbildungen ableiten lässt. Aus diesem Verhältnis wird ein eindeutiger numerischer Wert $\alpha$ abgeleitet.

Notwendigerweise muss die Korrelationsanalyse über einen vorgegebenen Zeitraum hinweg in regelmäßigen und zweckmäßigen Abständen wiederholt werden, um ein vollständiges Bild über eine zeitliche Entwicklung eines funktionellen Zustandes eines Organismus zu erhalten, wobei bei jedem Untersuchungstermin ein EKG aufgenommen, eine Korrelationsanalyse durchgeführt und ein aktueller diagnostischer Parameter $\alpha$ mit einem früheren diagnostischen Parameter $\alpha$ verglichen wird.

Eine weitere erfindungsgemäße Variante, welche sich ebenfalls auf eine Auswertung von Daten eines Elektrokardiogramms stützt, beruht auf dem Grundgedanken, mindestens abschnittsweise eine zeitliche Verlaufsform einer EKG-Kurve mittels eines Attraktormodells zu erfassen. Dazu wird ein Parametersatz in einem Anpassverfahren mindestens einer Modellfunktion solange verändert, bis diese Modellfunktion mindestens abschnittsweise den vorgegebenen EKG-Verlauf im Rahmen einer erforderlichen Genauigkeit reproduziert. Der Parametersatz, der in die Modellfunktion eingeht und zu dem das Anpassverfahren konvergiert, stellt somit einen Attraktor dieses Verfahrens dar, wobei sich gezeigt hat, dass die Gestalt dieses Attraktors signifikant mit einem Krebsrisiko des untersuchten Probanden korreliert ist.

Ein derartiges Auswerteverfahren berücksichtigt demnach die genaue Gestalt des EKG's an einer bestimmten Stelle selbst.

Es hat sich als zweckmäßig herausgestellt, als Modellfunktion mindestens ein Paar hinsichtlich ihrer Parametersätze gekoppelter Polynome n-ten Grades (n ganzzahlig, beliebig) zu verwenden, wobei bei steigendem Grad von n der EKG-Kurvenverlauf genauer reproduziert wird.

Bei Verwendung eines gekoppelten Paares von Polynomen mit der Zeit als unabhängige Variable ergibt das Attraktordiagramm in einer Parameterebene eine Kurve als Attraktor, die einen charakteristischen Krümmungsradius aufweist. Die Berechnung des Krümmungsradius an einer festgelegten Stelle liefert einen diagnostisch verwertbaren numerischen Wert. Für weitere detailliertere Ausführungen wird auf das Anwendungsbeispiel verwiesen.

Es ist zweckmäßig, den zeitlichen Kurvenverlauf des EKG an den Stellen des Q- und/oder S-Peaks in der beschriebenen Weise zu analysieren, wobei eine Standardabweichung eines Mittelwertes des zeitlichen Abstands zwischen den R-Peaks des EKG als Startwert für den iterativ zu bestimmenden Parametersatz eingeht.

Bei einer weiteren erfindungsgemäßen Variante des Auswerteverfahrens werden aus einer stetigen Zeitreihe von Intervallen quasiperiodischer EKG-Strukturen in einem Rhythmogramm auftretende relative Maxima des EKG-Verlaufs ausgewertet, eine Liste rhythmogrammbezogener Daten erzeugt und aus diesen Werten ein diagnostischer Wert ermittelt.

Im Gegensatz zu den voranstehend beschriebenen Auswerteverfahren wird hier die Aufmerksamkeit nicht auf vorliegende Korrelationen zwischen EKG-Verlaufsstrukturen oder den EKG-Verlauf selbst gelegt, sondern es wird das EKG auf rhythmische Muster überprüft. Dieses Verfahren untersucht somit die Gleichförmigkeit eines EKG nicht in Hinblick auf einen möglichen Zusammenhang zwischen einzelnen äquidistanten Stellen im EKG, sondern legt das Hauptaugenmerk auf den gesamten Herzrhythmus, der sich durch das EKG ausdrückt.

Im Einzelnen werden bei diesem Verfahren wieder die R-Peaks des EKG ausgewertet. Konkret werden die relativen Maxima der stetigen Zeitreihe der R-R-Intervalle in Abhängigkeit von einer Zahl der R-R-Abstände als Rhythmogramm ausgewertet. Die Zahl der R-R-Abstände wird gezählt und dient als unabhängige Variable des Rhythmogramms. Schlägt das Herz relativ gleichförmig, so entspricht jedem gezählten R-R-Abstand ein etwa gleichbleibender Abstand in der Zeitreihe der R-R-Intervalle und das Rhythmogramm enthält eine relativ gleichförmig aufeinanderfolgende Folge von Maxima und Minima. Bei einem ungleichförmig schlagenden Herzen schwankt die Zeitreihe der R-R-Intervalle in Abhängigkeit von der Zählzahl der R-R-Abstände stark. Dies schlägt sich im Rhythmogramm als eine unregelmäßige Folge von Maxima und Minima sowie einer zunehmenden Zahl von Sattelpunkten im Rhythmogramm nieder.

Im weiteren Auswerteverfahren werden die im so erhaltenen Rhythmogramm erfassten Messpunkte auf einen einheitlichen Zahlenwert normiert, wobei sich eine einheitliche Höhe der Rhythmogrammaxima ergibt.

Die Maxima des Rhythmogramms werden in folgender Weise ausgewertet:

Bei einem innerhalb eines Zählabstandes festgestellten Maximum im normierten Rhythmogramm wird diesem Abstand ein erster numerischer Wert beigelegt. Bei einem innerhalb eines Zählabstandes nicht festgestellten Maximum im normierten Rhythmogramm wird diesem Zählabstand ein zweiter numerischer Wert beigelegt. Das Ergebnis ist eine Zahlenreihe, die aus wechselnden numerischen Werten besteht. Über diese Zahlenreihe wird ein Mittelwert gebildet, der als diagnostisch verwertbare Größe dient.

Die erfindungsgemäßen Verfahren sollen nachfolgend anhand von Ausführungsbeispielen näher erläutert werden. Es werden für gleiche oder gleichwirkende Verfahrensschritte und Verfahrensbestandteile die selben Bezugsziffern verwendet. Zur Verdeutlichung dienen die nachfolgenden Figuren. Diese zeigen:

Fig. 1 ein schematisches Bild eines Kurvenverlaufs eines Elektrokardiogramms (EKG) nach dem Stand der Technik,

Fig. 2a, 2b ein Flußdiagramm eines ersten Ausführungsbeispiels des Korrelationsverfahrens,

Fig. 3a, 3b, 3c ein Flußdiagramm einer zweiten Ausführungsform des Korrelationsverfahrens,

Fig. 4a, 4b ein Flußdiagramm einer dritten Ausführungsform des Korrelationsverfahrens,

Fig. 5a, 5b ein Flussdiagramm einer vierten Ausführungsform des Korrelationsverfahrens,

Fig. 6a ein erstes beispielhaftes Korrelationsdiagramm mit einer ersten Punktwolkenform,

Fig. 6b ein zweites beispielhaftes Korrelationsdiagramm mit einer zweiten Punktwolkenform,

Fig. 7a ein erstes beispielhaftes Attraktordiagramm mit einem ersten Kurvenverlauf eines Attraktors,

Fig. 7b ein zweites beispielhaftes Attraktordiagramm mit einem zweiten Kurvenverlauf eines Attraktors,

Fig. 8a ein erstes beispielhaftes Rhythmogramm,

Fig. 8b ein normiertes Bild aller relativen Maxima des ersten beispielhaften Rhythmogramms aus Fig. 8a,

Fig. 9a ein zweites beispielhaftes Rhythmogramm,

Fig. 9b ein normiertes Bild aller relativen Maxima des zweiten beispielhaften Rhythmogramms aus Fig. 9a und

Fig. 10 ein Flussdiagramm zur Durchführung des Rhythmogrammverfahrens

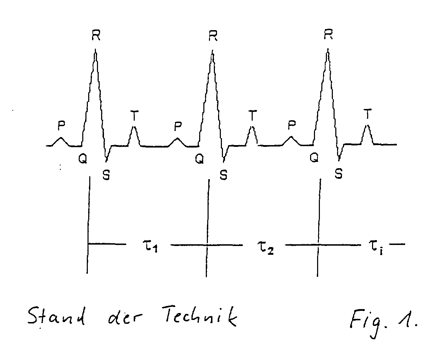

Fig. 1 zeigt in einer schematischen Darstellung eine typische EKG-Kurve, die den zeitlichen Verlauf eines elektrischen Potentials am aktiven menschlichen Herzen beschreibt. Eine solche Kurve ist durch charakteristische Peaks gekennzeichnet, die traditionell mit den Buchstaben P, Q, R, S und T gekennzeichnet werden. Dabei lassen sich diesen Strukturen eindeutig gewisse Herzaktivitäten zuordnen. Für die Beschreibung der nachfolgenden Anwendungsbeispiele sind besonders die systolischen R-Peaks und die negativen Q und S-Peaks von Bedeutung. Die zwischen den R-Peaks liegenden R-R-Zeitabstände sind in Fig. 1 mit $\tau_1$, $\tau_2$, $\tau_i$ bezeichnet.

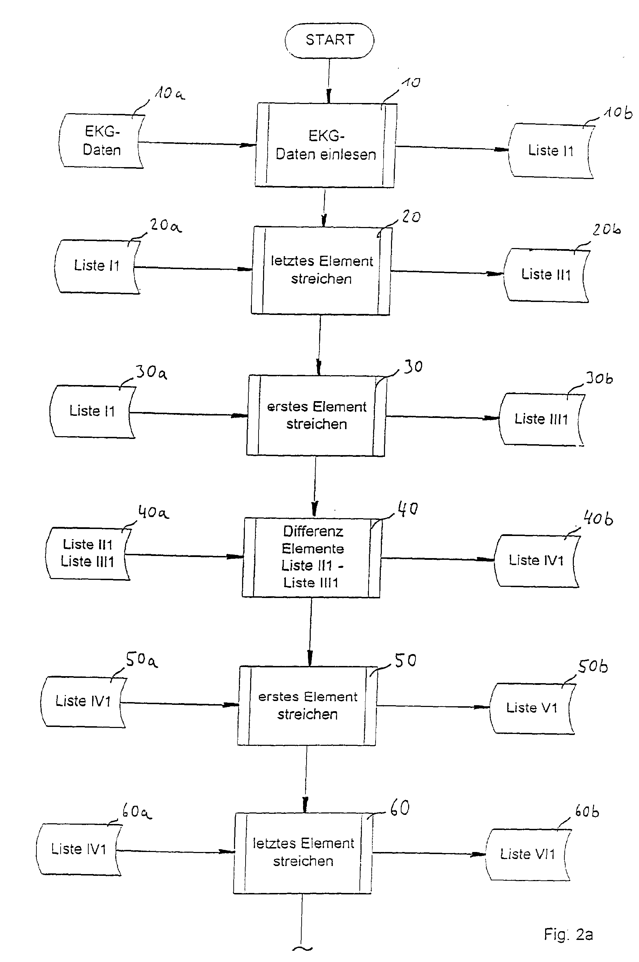

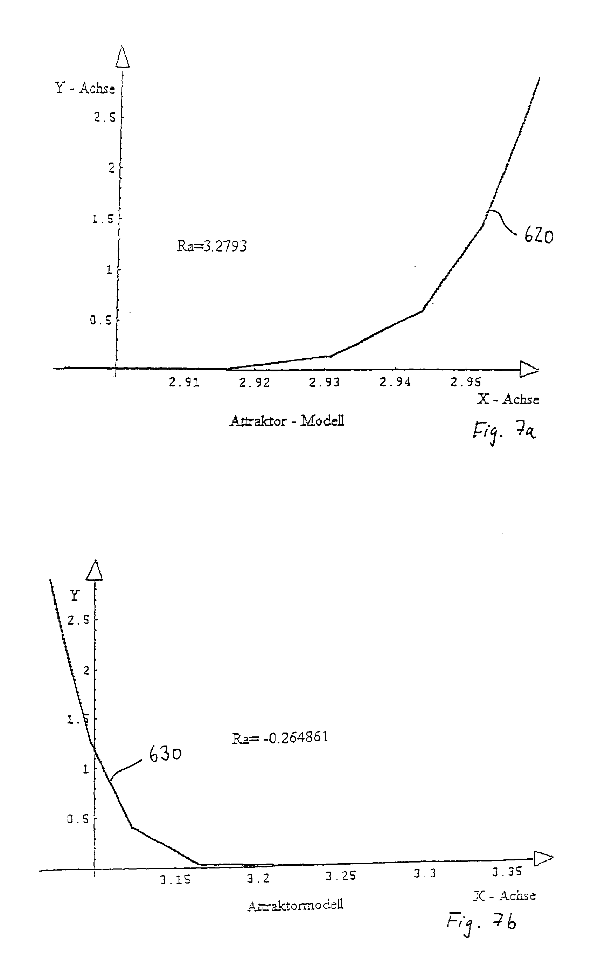

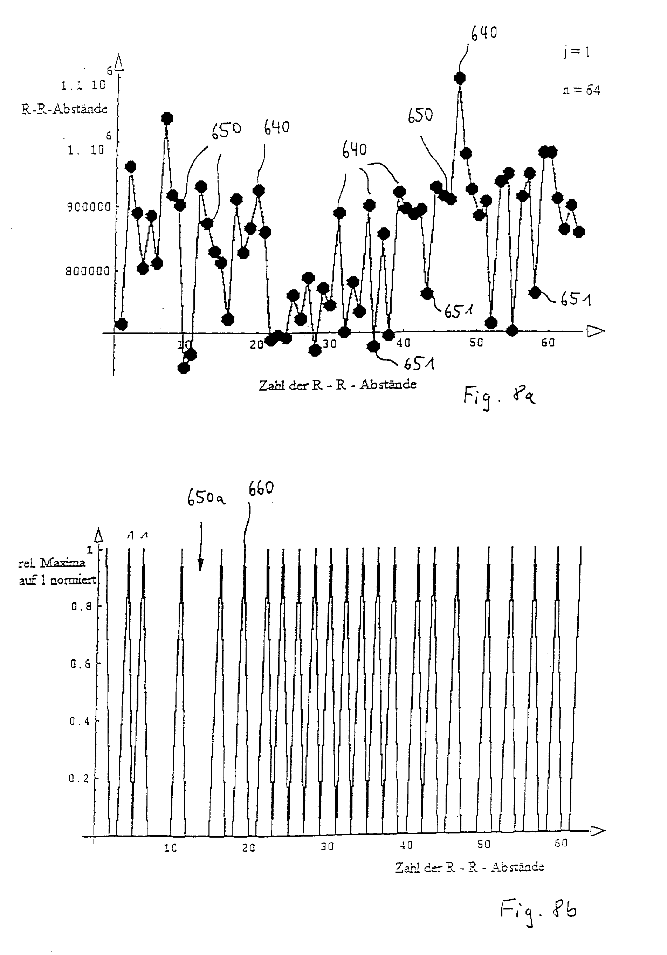

Die systolischen R-Peaks werden in den nachfolgenden Anwendungsbeispielen für die in den Figuren 2a bis 6b beschriebenen Korrelationsverfahren sowie die in den Figuren 8a bis 9b beschriebenen Rhythmogramm-Verfahren verwendet. Die Q- und S-Peaks sind Gegenstand des Attraktorverfahrens in den Figuren 7a und 7b.

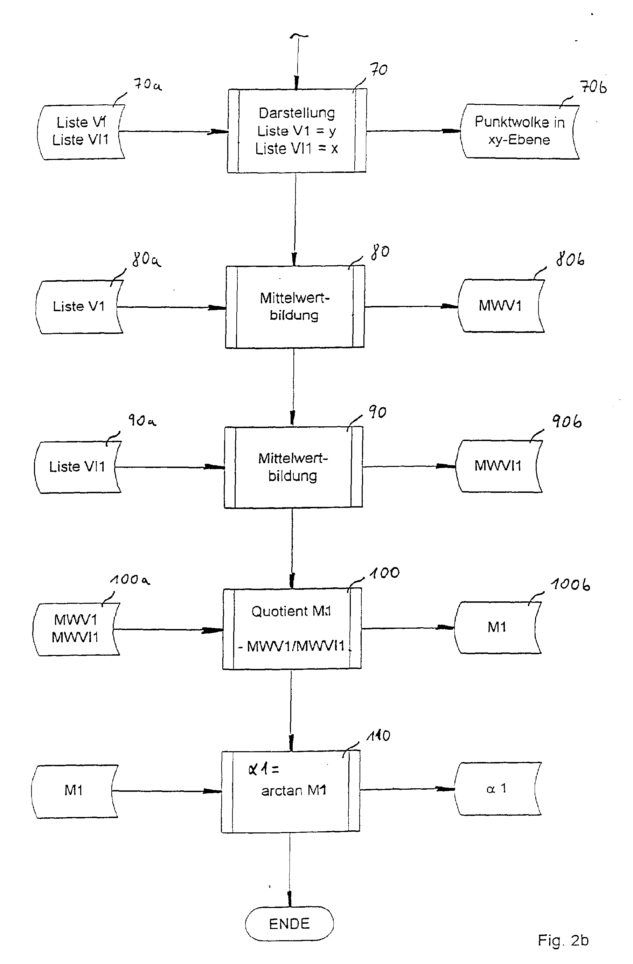

Die Figuren 2a und 2b stellen in einem Flussdiagramm beispielhaft einen einfachsten Ablauf eines Korrelationsverfahrens dar. Die Verfahrensschritte verlaufen in diesem aus Darstellungsgründen zweigeteilten Diagramm in senkrechter Richtung, während die während des Verfahrens erzeugten Ausgaben jeweils rechts dargestellt und die benötigten Eingaben links angeordnet sind. Hinsichtlich der Bezugszeichen erhalten alle Verfahrensschritte durch zehn teilbare Bezugsziffern, wobei eine Eingabe eines Verfahrensschrittes eine mit einem "a" ergänzte Bezugsziffer des Verfahrensschrittes und eine Ausgabe des Verfahrensschrittes eine mit einem "b" ergänzte Bezugsziffer erhält. Die verarbeiteten Daten tragen Buchstabenbezeichnungen.

Bei dem in den Figuren 2a und 2b dargestellten Verfahren zur Korrelationsanalyse werden bei einem ersten Einlesen 10 eine Liste von zeitlichen R-R-Intervallen aus Daten 10a einem Elektrokardiogramm in einen Speicher übertragen. Liegen die zeitlichen R-R-Intervalle im Elektrokardiogramm noch nicht vor, werden diese aus der Differenz der absoluten Zeitpunkte aufeinander folgender R-Peaks errechnet Es entsteht eine Liste I1 10b, welche fortlaufend zeitliche R-R-Intervalle beinhaltet. Diese Liste kann an sich beliebig viele Daten enthalten. Aus Zweckmäßigkeitsgründen für spätere Verfahrensschritte und weitere Verfahrensvarianten wird im Folgenden davon ausgegangen, dass die Liste I1 genau 512 Elemente enthält.

Die Liste I1 geht als Eingabe 20a in einen Verfahrensschritt 20 ein, bei dem das letzte Element der Liste I1 gestrichen wird. Es erfolgt eine Ausgabe 20b einer Liste II1. Ebenfalls geht die Liste I1 als Eingabe 30a in einen Verfahrensschritt 30 ein, bei dem das erste Element der Liste I1 gestrichen wird. Es erfolgt eine Ausgabe 30b einer Liste III1.

Die Listen II1 und III1 gehen beide als eine Eingabe 40a in einen Verfahrensschritt 40 ein, in welchem eine Differenzbildung der Elemente der Liste II1 von den Elementen der Liste III1 erfolgt. Das Ergebnis wird als eine Ausgabe 40b in einer Liste IV1 abgelegt.

Die Elemente der Liste IV1 gehen als eine Eingabe 50a in einen Verfahrensschritt 50 ein, in welchem das erste Element der Liste IV1 gestrichen wird. Das Ergebnis wird als eine Ausgabe 50b in eine Liste V1 überführt. Die Elemente der Liste IV1 gehen darüber hinaus als eine Eingabe 60a in einen Verfahrensschritt 60 ein, bei welchem das letzte Element der Liste IV1 gestrichen wird. Das Ergebnis wird als eine Ausgabe 60b in einer Liste VI1 abgelegt.

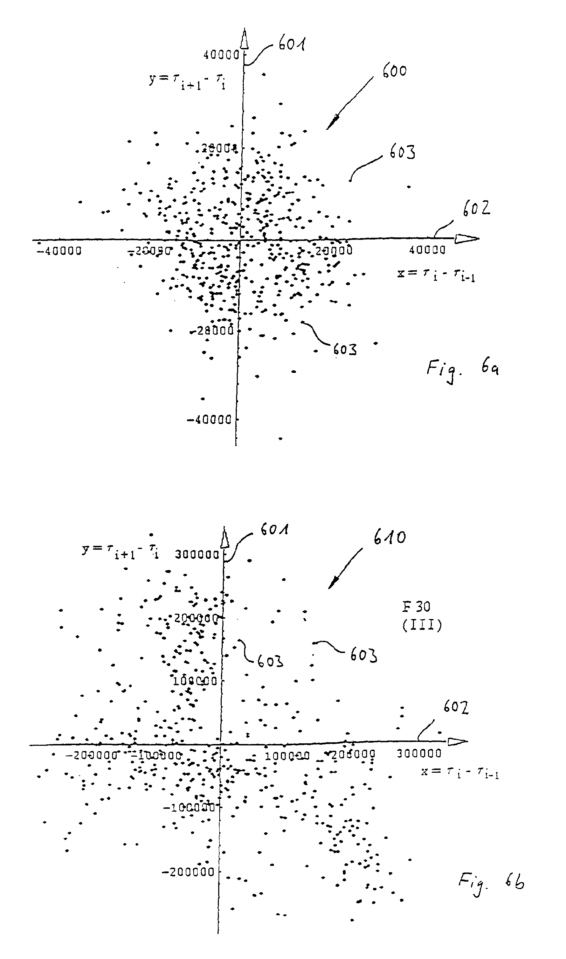

Die Listen V1 und VI1 dienen als Eingabewerte 70a in einem Verfahrensschritt 70 zum Erzeugen einer graphischen Darstellung, wobei die Elemente der Liste V1 als Ordinate über den Elementen der Liste V1 als Abszisse dargestellt werden. Das Ergebnis ist eine Punktwolke in einer xy-Ebene. Ein Beispiel einer solchen Punktwolke ist in den Figuren 6a und 6b dargestellt. Beispielhaft ist hier eine erste Differenzbildung $\tau_{i+1}-\tau_i$ eines Nachfolge-R-R-Zeitintervalls über einer zweiten Differenzbildung $\tau_i-\tau_{i-1}$ eines Vorgänger R-R-Zeitintervalls dargestellt, wobei sich zeigt, dass die so dargestellte Punktwolke 600 charakteristische Formen annehmen kann.

Zur Berechnung eines diagnostisch aussagekräftigen numerischen Wertes erfolgen Eingaben 80a und 90a zu Mittelwertbildungen 80 und 90, wobei die Mittelwerte vorzeichenrichtig ermittelt werden. Es erfolgen Ausgaben 80b und 90b von Mittelwerten MWV1 und MWVI1.

Zu einer Quotientenberechnung 100 werden beide Mittelwerte MWV1 und MWVI1 in einem Eingabeschritt 100a eingelesen und es erfolgt eine Quotientenbildung M1 = - MWV1/MWVI1, wobei der Quotient M1 in einem Ausgabeschritt 100b ausgegeben wird. Der diagnostische numerische Wert wird nachfolgend durch eine Eingabe 110a in einen Berechnungsschritt 110 errechnet, wobei ein diagnostischer Parameter $\alpha$ = arctan M gebildet und in einem Ausgabeschritt 110b ausgegeben wird.

Es hat sich gezeigt, dass der so gewonnene Wert $\alpha$ mit dem onkologischen Risiko des untersuchten Patienten korreliert. Durch Testreihen an Elektrokardiogrammen von 110 Personen, für die eine ärztliche onkologische Beurteilung vorlag, konnte festgestellt werden, dass mit dieser Methode der Korrelationsanalyse eine mehr als 50-prozentige Übereinstimmung mit den vorliegenden ärztlichen Einschätzungen erzielt wurde, wenn dem numerischen Wert $\alpha$ folgende diagnostische Bewertung zugeordnet wurde:

- 120° < $\alpha$ < 180° und 0° < $\alpha$ < 20°: onkologisch unbedenklich;
- 80° < $\alpha$ < 120°: Untersuchung nach 3 Monaten wiederholen;
- 20° < $\alpha$ < 80°: hohes onkologisches Risiko, eingehende onkologische Untersuchung notwendig.

Eine in den Figuren 2a und 2b beschriebene Verfahrensweise stellt eine Langzeitkorrelation zwischen den jeweils ersten beiden und letzten beiden R-R-Abständen $\tau i$ eines Elektrokardiogramms dar.

Im folgenden wird auf weitere Korrelationsverfahren zur Auswertung der R-R-Zeitintervalle eingegangen.

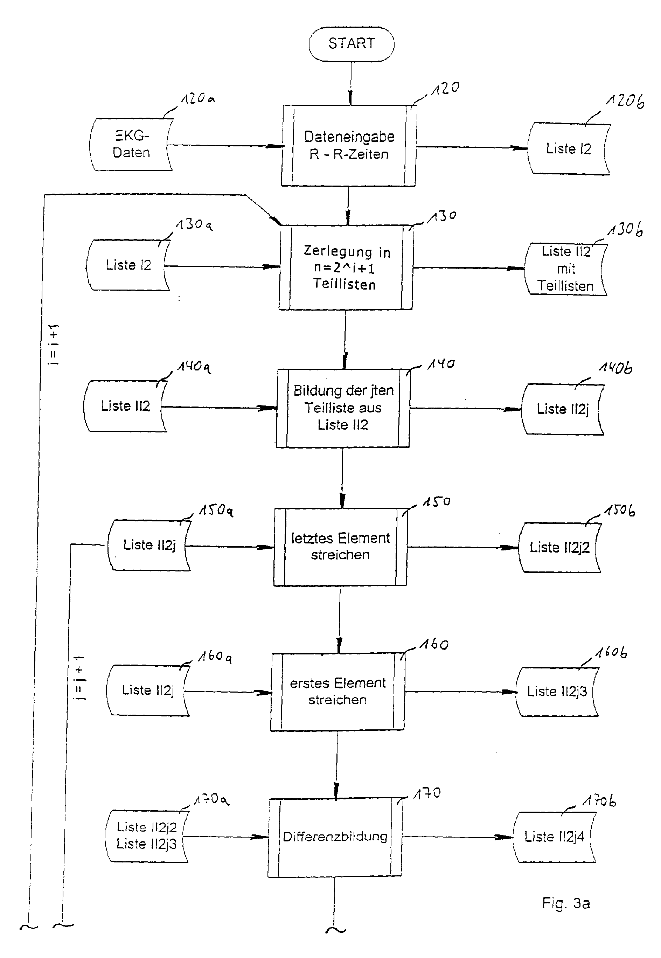

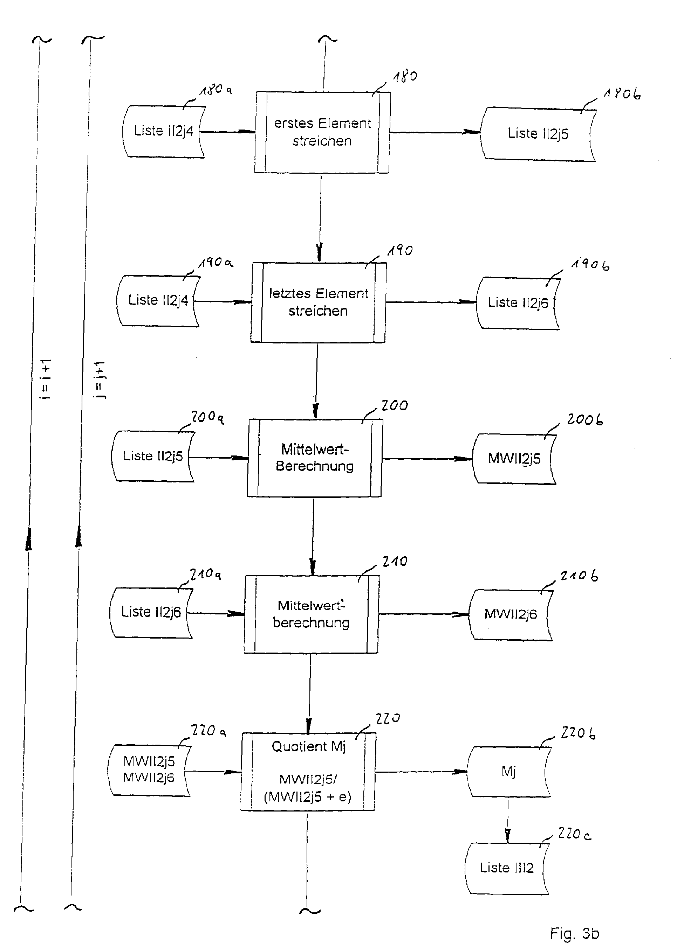

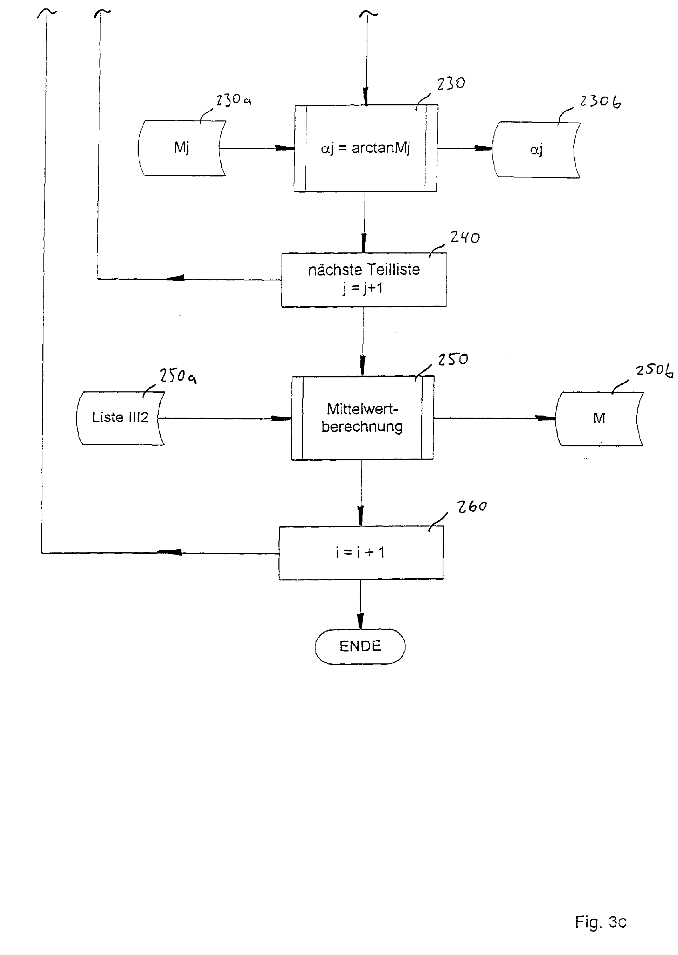

Dem in den Figuren 3a bis 3c dargestellten beispielhaften Verfahrensablauf liegt die fortbildende Idee zugrunde, die im ursprünglichen EKG gegebene Liste der R-R-Zeitintervalle τi in Teillisten zu zerlegen und über jede Teilliste gesonderte Mittelwerte MW, Quotienten M und diagnostische Werte α zu berechnen. Dieser Verfahrensablauf stellt eine Möglichkeit bereit, Kurzzeitkorrelationen in einer Folge von R-R-Zeitintervallen abgestuft und mit veränderlicher Feinheit zu untersuchen.

Diese Ausführungsform der Korrelationsanalyse beginnt mit einem Einlesevorgang 120 aller R-R-Zeitintervalle τi, wobei in einem Ausgabeschritt 120b eine Liste I2 erzeugt wird. Die Elemente der Liste I2 werden als Eingabe 130a einem Zerlegungsschritt 130 zugeführt, welcher die Liste I2 in $n = 2^{(i+1)}$ Teillisten zerlegt. Dabei stellt i einen verfahrensinternen Laufindex dar, der zunächst als $i = 1$ gesetzt wird. Es wird nachfolgend in einem Ausgabeschritt 130b eine Liste II2 erzeugt, die aus n Teillisten besteht.

Die Liste II2 geht als Eingabe 140a in einen Verfahrensschritt 140 ein, in welchem aus der Liste II2 eine j-te Teilliste ausgewählt wird. Der Index j bezeichnet auch hier einen verfahrensinternen Laufindex, wobei zunächst $j = 1$ gesetzt wird. In einem Ausgabeschritt 140b wird als Resultat dieses Verfahrensschrittes 140 eine Liste II2j erzeugt.

Die Liste II2j wird als Eingabewerte-Tabelle 150a einem Verfahrensschritt 150 zugeführt, bei welchem das letzte Element dieser Teilliste gestrichen wird. In einem Ausgabeschritt 150b entsteht so eine Liste II2j2. Die Liste II2j wird als Eingabe 160a einem weiteren Verfahrensschritt 160 zugeführt, in welchem das erste Element der Liste gestrichen wird. Es wird in einem Ausgabeschritt 160b eine Liste II2j3 erzeugt.

Beide Listen II2j2 und II2j3 werden als Eingabedaten 170a für eine Differenzbildung 170 bereitgestellt, wobei in einem Ausgabeschritt 170b eine Liste II2j4 erzeugt wird.

Die Elemente der Liste II2j4 wird als Eingabe 180a einem Verfahrensschritt 180 zugeführt, in welchem das erste Element der Liste II2j4 gestrichen wird. Es wird anschließend in einer Ausgabe 180b eine Liste II2j5 erzeugt. Die Liste II2j4 wird als Eingabe 190a einem Verfahrensschritt 190 zugeführt, in welchem das letzte Element der Liste II2j4 gestrichen wird. Es wird in einem Ausgabeschritt 190b eine Liste II2j6 erzeugt.

Von den Listen II2j5 und II2j6, die als Eingaben 200a und 210a Mittelwertberechnungen 200 und 210 zugeführt werden, werden vorzeichenrichtige Mittelwerte MWII2j5 und MWII2j6 errechnet und als Ausgaben 200b und 210b ausgegeben.

Beide Mittelwerte MWII2j5 und MWII2j6 gehen als Werte einer Eingabe 220a für eine Quotientenberechnung 220 ein, in der ein Teillistenmittelwert Mj nach folgender Berechnungsformel errechnet wird: -MWII2j6/(MWII2j5 + e), wobei e eine Korrekturvariable ist, die eine Division durch Null vermeidet. e ist entsprechend zu wählen. Der Quotient Mj wird als Ausgabe 220b und 220c in eine Liste III2 übertragen.

Auch hier kann ein teillistenbezogener diagnostischer Wert αj aus dem Quotienten Mj nach der bereits beschriebenen Berechnungsformel $αj = \arctan Mj$ errechnet werden. Mj wird dazu als eine Eingabe 230a in einen Berechnungsschritt 230 eingelesen, wobei die diagnostischen Größen αj in einer Ausgabe 230b ausgegeben werden. Auch diese können gegebenenfalls in einer Liste erfasst werden.

Nach Abschluß dieser Prozedur wird der Laufindex j in einem Zählschritt 240 um 1 erhöht und es erfolgt die Verarbeitung einer nächsten Teilliste an Verfahrensschritt 150 mit einer Eingabe 150a der j+1ten Teilliste II2j+1.

Es wird somit die ursprüngliche Liste in Teillisten zerlegt, wobei innerhalb der Teillisten gesonderte Korrelationsverfahren angewendet werden. Diese Ausführungsform des Korrelationsverfahrens kann um einen Verfahrensschritt 250 ergänzt werden, wobei aus der Liste III2 der Quotienten Mj ein weiterer Mittelwert M gebildet wird. Die Liste III2 wird als Eingabe 250a in einen Verfahrensschritt 250 eingelesen, wobei eine Ausgabe 250b des Gesamtmittelwertes M erfolgt.

Weiterhin kann das Verfahren noch um einen Verfahrensschritt 260 erweitert werden, in welchem der eingangs erwähnte Laufindex i um 1 auf i+1 erhöht wird. Die so veränderte Größe des Laufindex i wird auf den Verfahrensschritt 130 rückgekoppelt, wobei sich die Zerlegung der ursprünglichen Liste I2 in die Teillisten innerhalb der Liste II2 in Potenzen von 2 verfeinert.

Diese verallgemeinerte Korrelationsanalyse erlaubt es, das vorliegende Material der R-R-Zeitintervalle mit einer abgestimmten Feinheit zu untersuchen. Es zeigte sich, dass mit einer Zerlegung von vorgegebenen 512 R-R-Intervallen in Teillisten zu je 64 Elementen eine etwa 60-prozentige Übereinstimmung der unter Verwendung dieser Korrelationsanalyse erzielten onkologischen Prognose mit bereits bekannten onkologischen Untersuchungsdaten erreicht werden konnte, wobei die gleiche Probandengruppe von 110 Personen überprüft wurde.

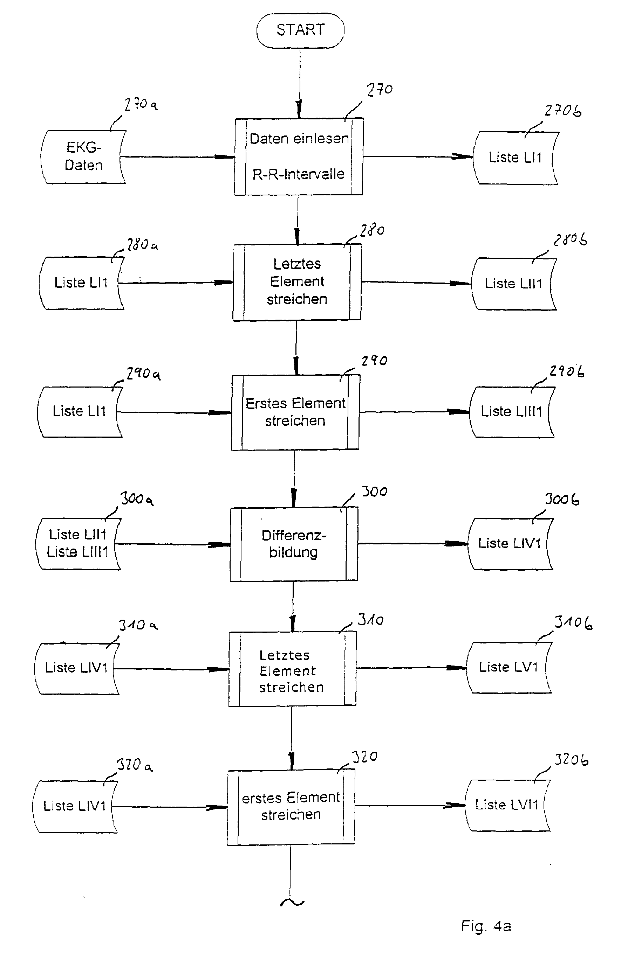

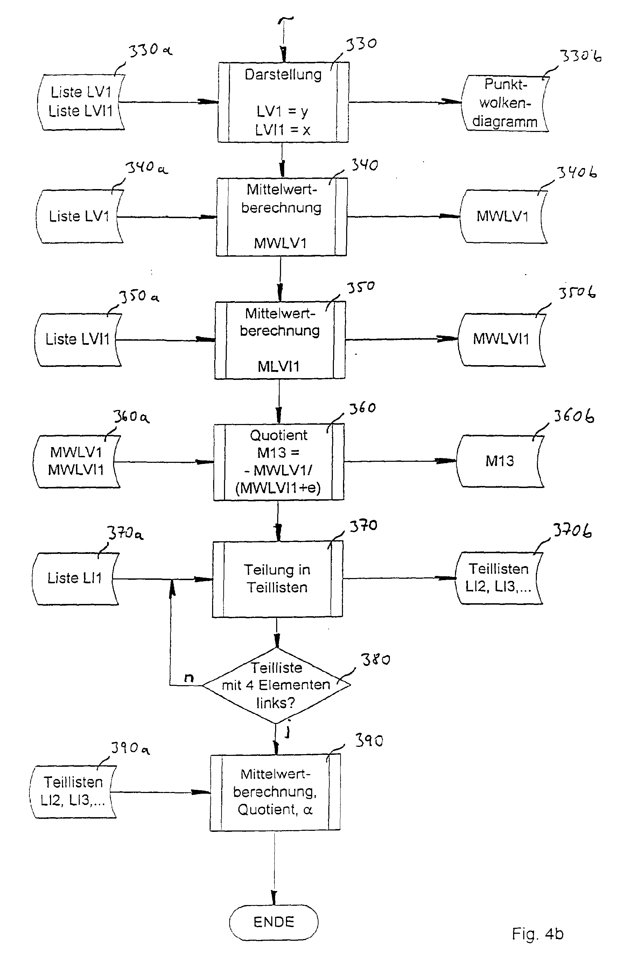

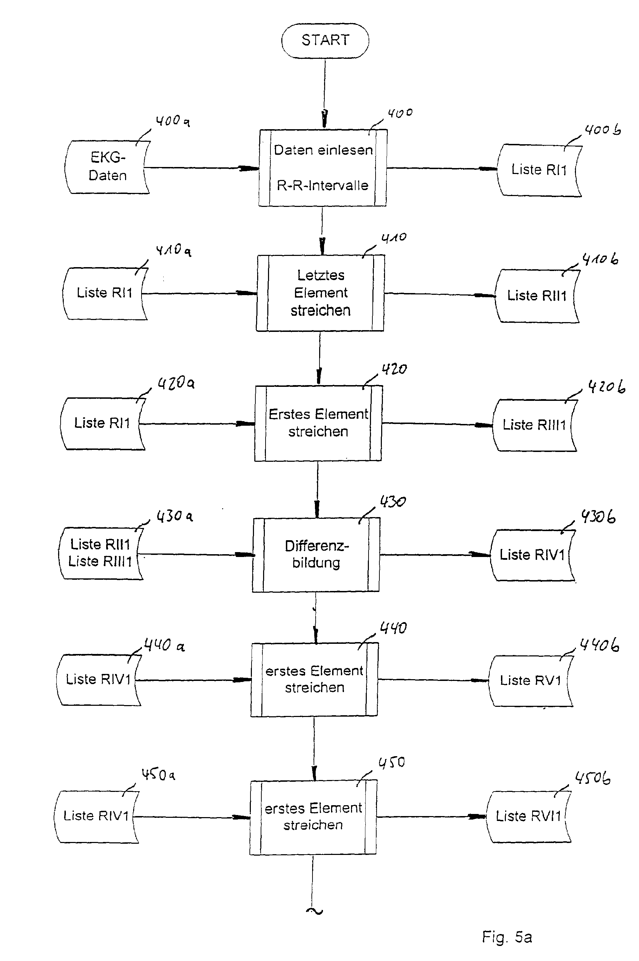

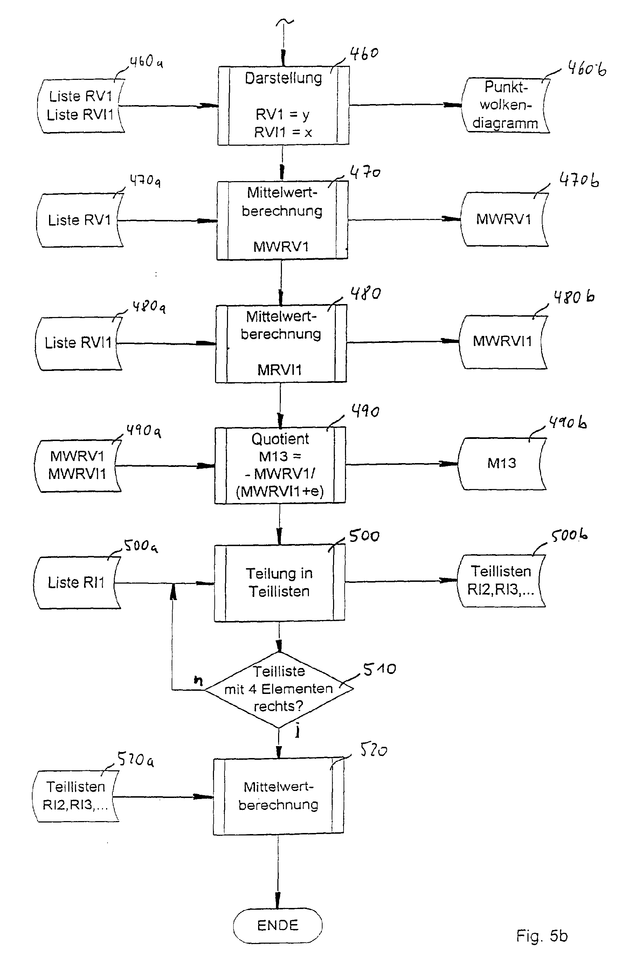

Im folgenden werden Korrelationsanalysen beschrieben, die eine besondere Gewichtung der Reihe der R-R-Zeitintervalle nach einem Anfang und einem Ende vornehmen. Flussdiagramme zur Ausführung derartiger Korrelationsanalysen sind in den Figuren 4a und 4b einerseits und in den Figuren 5a und 5b andererseits dargestellt und werden nachfolgend anhand dieser Figuren erläutert. Die im folgenden genannten Begriffe "linksseitig" und "rechtsseitig" beziehen sich auf den Anfang bzw. das Ende der ursprünglichen Reihe der R-R-Zeitintervalle.

Zunächst wird im Flussdiagramm nach den Figuren 4a und 4b auf eine Anfangskorrelationsanalyse eingegangen. Dieses Verfahren beginnt ebenfalls mit einem Einlesen 270 der Reihe der R-R-Zeitintervalle, wobei in einem Ausgabeschritt 270b eine Liste LI1 erzeugt wird. Die Liste LI1 wird nach dem bereits beschriebenen Korrelationsverfahren innerhalb der Verarbeitungsschritte 280 bis 350 ausgewertet, wobei in einem Verfahrensschritt 360 ein Quotient M13 in einem Ausgabeschritt 360b für die gesamte Liste LI1 ausgegeben wird, wobei ebenfalls eine diagnostische Größe αI13 berechnet werden kann. Die Korrelationsanalyse wird aber um folgende Schritte ergänzt:

Die Liste LI1 wird als Eingabe 370a einem Verfahrensschritt 370 zugeführt, wobei dieser eine Teilung in Teillisten LI2, LI3,... ausführt. Dabei wird jeweils die linksseitige Teilliste solange weiter unterteilt, bis in einem Entscheidungsschritt 380 das Vorliegen einer linksseitigen Teilliste mit einer gegebenen Anzahl von R-R-Zeitintervallen festgestellt worden ist. Im in Fig. 4b dargestellten Beispiel umfasst die linksseitige Teilliste beispielhaft 4 Elemente, d.h. 4 R-R-Zeitintervalle. Es liegen somit nach Abschluß der Verfahrensschritte 370 und 380 linksseitig kumulierte Teillisten vor, die einzeln mit dem bereits beschriebenen Korrelationsanalyseverfahren bearbeitet werden können, wobei sowohl eine Analyse einer anfänglichen, kurzzeitigen Korrelation und langzeitigen Korrelation mit gewissen Zwischenstufen möglich ist. Dieses Auswerteverfahren erlaubt es somit, anfangsbezogene, zeitabhängige Korrelationsanalysen von R-R-Zeitintervallen auszuführen.

Analog dazu erfolgt eine Endkorrelationsanalyse nach den Flussdiagrammen der Figuren 5a und 5b. Die dort dargestellten Verfahrensschritte 400 bis 490 mit den Eingaben 410a bis 490a und den zugeordneten Ausgaben 410b bis 490b erfolgen analog zur anfänglich beschriebenen Korrelationsanalyse. Die Teilung 500 in Teillisten RI2, RI3,... erfolgt analog zu den in Fig. 4b dargestellten Verfahrensschritten 370 und 380, allerdings mit dem Unterschied, dass innerhalb einer Entscheidungsprozedur 510 eine Abfrage nach einer Anzahl von Elementen einer rechten Teilliste erfolgt. Mittels dieser endbezogenen Korrelationsanalyse ist eine zeitliche Entwicklung einer Korrelation innerhalb der Reihe der R-R-Zeitintervalle gegen ein Ende dieser Reihe möglich.

Wie bereits erwähnt, werden mittels dieser Korrelationsanalysen Punktwolken 600 und 610 in einem zweidimensionalen Korrelationsdiagramm erzeugt. In den Figuren 6a und 6b sind zwei Beispiele solcher Korrelationsdiagramme dargestellt. Diese zeigen jeweils eine erste Reihe von Differenzbildungen $\tau_{i+1} - \tau_i$, aufgetragen an Ordinaten 601 und eine zweite Reihe von Differenzbildungen $\tau_i - \tau_{i-1}$, aufgetragen an Ordinaten 602. Die einzelnen Punkte 603 der Punktwolken 600 und 610 entstehen durch Zuordnung der Elemente der Reihe der ersten Differenzbildungen zu den Elementen der Reihe der zweiten Differenzbildungen. Durch einen Vergleich der Form der Punktwolke 600 aus Fig. 6a mit der Form der Punktwolke 610 aus Fig. 6b geht hervor, dass die Punkte 603 der Punktwolke 600 in einem relativ eng begrenzten Bereich konzentriert sind, während die Punkte 603 der Punktwolke 610 über einen weiten Bereich gestreut sind.

Eine Punktwolke 600 in einer konzentrierten Form lässt einen Rückschluß auf eine regelmäßige Herztätigkeit zu, bei der die R-R-Zeitintervalle offensichtlich in einem begrenzten Bereich korreliert sind, während die Punktwolke 610 ein weniger korreliertes Verhalten der R-R-Zeitintervalle zueinander offenbart. Derartige Punktwolkenformen 610 lassen auf eine in ihrem "Takt" gestörte Herztätigkeit schließen.

Im Folgenden wird unter Bezugnahme auf die Figuren 7a und 7b ein Anwendungsbeispiel des Attraktormodells näher erläutert. Grundlage dafür sind Verhaltensweisen des EKG-Verlaufs an den Stellen der Q- und S-Peaks im Elektrokardiogramm. Diese werden durch Modellfunktionen angepasst, wobei deren Parameter ein Attraktordiagramm ergeben.

Bewährt haben sich als Modellfunktionen ein Paar gekoppelte Funktionen zweiten Grades

$$X = X_0 + (X_0^3 \cdot t - 3 \cdot X_0^2 \cdot t) \cdot (X_0^2 \cdot t + Y_0^2 \cdot t)^{0.5} + \lambda \cdot X_0 \cdot t \cdot (X_0^2 \cdot t + Y_0^2 \cdot t - 1) + \mu \cdot X_0 \cdot t \cdot (X_0^2 \cdot t + Y_0^2 \cdot t)^{0.5}$$

$$Y = Y_0 + (Y_0^3 \cdot t - 3 \cdot X_0^2 \cdot Y_0 \cdot t) \cdot (X_0^2 \cdot t + Y_0^2 \cdot t)^{0.5} + \lambda \cdot Y_0 \cdot t \cdot (X_0^2 \cdot t + Y_0^2 \cdot t - 1) + \mu \cdot X_0 \cdot t \cdot (X_0^2 \cdot t + Y_0^2 \cdot t)^{0.5},$$

wobei iterativ die Parameter X und Y berechnet werden, die anschließend wieder als Startwerte für weitere Iterationsschritte dienen. Es wird mit den Startwerten $X_0$ und $Y_0$ begonnen, wobei bei der Wahl des Startwertes $X_0$ die Standardabweichung und der Mittelwert einer großen Anzahl von R-R-Intervallen, beispielsweise von 512 R-R-Intervallen, einbezogen werden. Nach einer gewissen Anzahl von Iterationsschritten konvergieren die Parameter beider Polynome gegen gewisse Werte, die in einem zweidimensionalen Diagramm ein graphisches Objekt bilden, was als Attraktor bezeichnet wird.

In den Figuren 7a und 7b sind beispielhaft zwei Attraktorkurven 620 und 630 dargestellt. Die Attraktorkurve 620 zeigt einen Verlauf, dem ein positiver Krümmungsradius zuzuordnen ist, wobei dieser in diesem Anwendungsbeispiel etwa R = 3.3 beträgt. Dagegen zeigt die Attraktorkurve 630 einen Verlauf mit einem negativen Krümmungsradius, der in diesem Beispiel einen Wert von etwa R = -0.3 annimmt. Die Attraktorkurve 620 ist einem onkologisch ungefährdeten Patienten zugeordnet, während die Kurve 630 einem aus onkologisch stark gefährdeten Patienten zugeordnet ist, dessen onkologische Gefährdung durch weitere diagnostische Methoden bestätigt wurde. Sowohl der Absolutbetrag des Krümmungsradius als auch dessen Vorzeichen dienen als Klassifizierungsmerkmal für das Vorliegen einer onkologischen Gefährdung, also eines Krebsrisikos.

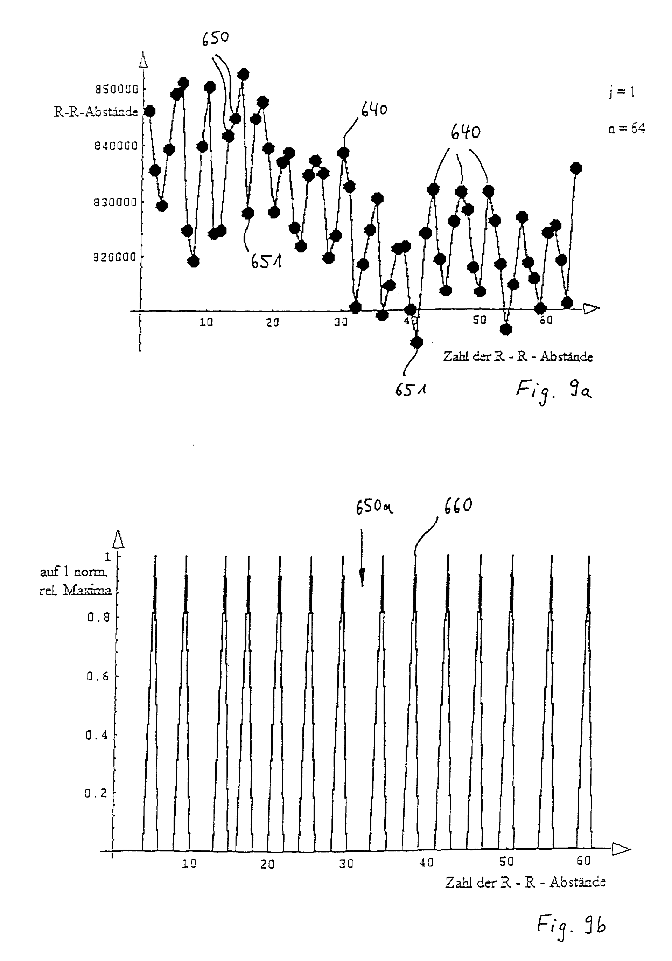

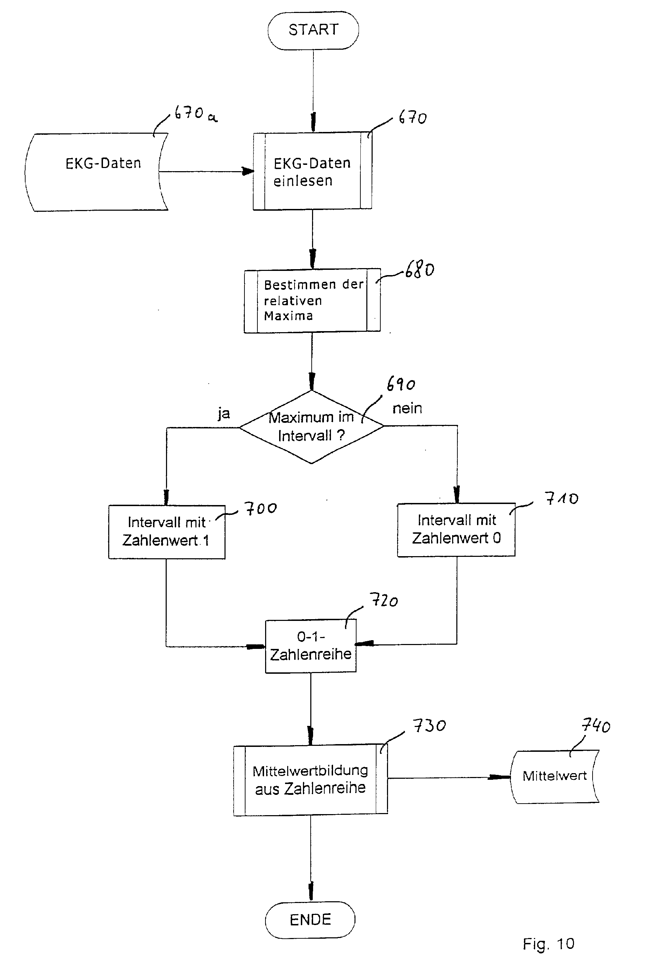

In den Figuren 8a,b sowie in den Figuren 9a,b als auch in dem in der Figur 10 dargestellten Flussdiagramm wird die Methode der relativen Maxima eines Rhythmogramms anhand von Anwendungsbeispielen näher erläutert.

Ein Rhythmogramm ist die Darstellung der Zeitintervalle zwischen gleichartigen Ereignissen über der Zählreihe der Ereignisse. Im hier vorliegenden Anwendungsbeispiel werden als Ereignisse die systolischen R-Peaks verwendet, wobei die sich zwischen ihnen erstreckenden Zeitintervalle und deren Zahl aus der Zeitskala, bzw. der Anzahl der R-Peaks eines Elektrokardiogramms ermittelt werden. Beispiele für ein Rhythmogramm sind jeweils in den Figuren 8a und 9a dargestellt. Die Datenpunkte innerhalb eines Rhythmogramms werden dabei hinsichtlich ihrer Lage klassifiziert. Bei einer ersten Art von Datenpunkten beschreibt die Lage des betreffenden Punktes im Rhythmogramm ein relatives Maximum 640, während bei einer zweiten Art von Datenpunkten der Punkt einen Sattel 650 oder ein relatives Minimum 651 beschreibt.

Erfindungsgemäß werden nun aus den in den Figuren 8a und 9a dargestellten Rhythmogrammen alle diejenigen Datenpunkte extrahiert, die ein relatives Maximum innerhalb des Rhythmogramms beschreiben, während alle weiteren Datenpunkte ausgesondert werden.

Eine normierte Darstellung aller relativer Maxima 640 ist jeweils in den Figuren 8b und 9b dargestellt. Es ergibt sich ein Bild aufeinander folgender Zacken 660, wobei durch die Aussonderung von Sattelpunkten 650 und Punkten relativer Minima 651 einigen Zählintervallen der R-R-Abstände kein Punkt zugeordnet ist. Die normierten und extrahierten Rhythmogramme in den Figuren 8b und 9b weisen an solchen Stellen Lücken 650a auf, die besonders in Fig. 8b gut zu erkennen sind.

Jedes Zählintervall der Zählreihe der R-R-Abstände ist eindeutig durch eine Zacke oder das Fehlen einer Zacke gekennzeichnet. In der weiteren Auswertung wird die Zackenstruktur innerhalb der Zählreihe analysiert. Ein Flussdiagramm einer solchen Auswertung ist in Fig. 10 dargestellt.

Ausgangspunkt des Auswertevorgangs sind die EKG-Daten. In einem ersten Verarbeitungsschritt 670 werden die EKG-Daten eingelesen und ein Rhythmogramm erstellt. In einem Verarbeitungsschritt 680 wird die Lage der relativen Maxima innerhalb der Zählreihe der R-R-Abstände bestimmt. Dann folgt eine Entscheidung 690 darüber, ob einem Glied der Zählreihe der R-R-Intervalle ein normiertes Maximum zugeordnet werden kann, oder nicht. Fällt die Entscheidung positiv aus, wird diesem Zählintervall in einem Schritt 700 der Zahlenwert 1 beigelegt, anderenfalls erhält dieses Zählintervall in einem Alternativschritt 710 den Wert 0.

Das Ergebnis dieser Operationen ist eine 0-1-Zahlenreihe 720, deren Mittelwert 740 als numerischer und diagnostischer Wert dient.

Beispielsweise ergeben die Rhythmogrammdaten eines onkologisch gefährdeten Probanden in den Figuren 8a und 8b einen Mittelwert von etwa 0.4, während der aus dem Rhythmogrammen eines onkologisch ungefährdeten Probanden in den Figuren 9a und 9b errechnete Mittelwert bei etwa 0.2 liegt. In Testreihen wurden mit der Rhythmogrammmethode Übereinstimmungen mit den Ergebnissen weiterer onkologischer Nachweismethoden von mehr als 70 Prozent erzielt.

Bezugszeichenliste

| | |
|---|---|
| 10 | Einlesen der EKG-Daten |
| 10a | Eingabe: EKG-Daten |
| 10b | Ausgabe: Liste I1 |
| 20 | letztes Element streichen |
| 20a | Eingabe: Liste I1 |
| 20b | Ausgabe: Liste II1 |
| 30 | erstes Element streichen |
| 30a | Eingabe: Liste I1 |
| 30b | Ausgabe: Liste III1 |
| 40 | Differenzbildung |
| 40a | Eingabe: Elemente der Listen II1 und III1 |
| 40b | Ausgabe: Liste IV1 |
| 50 | erstes Element streichen |
| 50a | Eingabe: Liste IV1 |
| 50b | Ausgabe: Liste V1 |
| 60 | letztes Element streichen |
| 60a | Eingabe: Liste IV1 |
| 60b | Ausgabe: Liste VI1 |

| 70 | Darstellung: Elemente der Liste V1 über Elemente der Liste VI1 |
|---|---|
| 70a | Eingabe: Liste VI1, Liste V1 |
| 70b | Ausgabe: Punktwolkendiagramm |
| 80 | Mittelwertbildung, vorzeichengerecht |
| 80a | Eingabe: Liste V1 |
| 80b | Ausgabe: Mittelwert MWV1 |
| 90 | Mittelwertbildung, vorzeichengerecht |
| 90a | Eingabe: Liste VI1 |
| 90b | Ausgabe: Mittelwert MWVI1 |
| 100 | Quotientenbildung |
| 100a | Eingabe: Mittelwerte MWV1, MWVI1 |
| 100b | Ausgabe: numerischer Wert M1 |
| 110 | Berechnung $\alpha 1$ = arctan M1 |
| 110a | Eingabe: M1 |
| 110b | Ausgabe: $\alpha 1$ |
| 120 | Dateneingabe: R-R-Zeiten |
| 120a | Eingabe: EKG-Daten |
| 120b | Ausgabe: Liste I2 |
| 130 | Zerlegung in n = 2i Teillisten |
| 130a | Eingabe: Liste I2 |
| 130b | Ausgabe: Liste II2, aus 2i Teillisten bestehend |
| 140 | Bildung der jten Teilliste aus Liste II2 |
| 140a | Eingabe: Liste II2 |
| 140b | Ausgabe: Liste II2j |
| 150 | letztes Element streichen |
| 150a | Eingabe: Liste II2j |
| 150b | Ausgabe: Liste II2j2 |
| 160 | erstes Element streichen |
| 160a | Eingabe: Liste II2j |
| 160b | Ausgabe: Liste II2j3 |
| 170 | Differenzbildung |
| 170a | Eingabe: Listen II2j2 und II2j3 |
| 170b | Ausgabe: Liste II2j4 |
| 180 | erstes Element streichen |
| 180a | Eingabe: Liste II2j4 |
| 180b | Ausgabe: |
| 190 | letztes Element streichen |
| 190a | Eingabe: Liste II2j4 |
| 190b | Ausgabe: Liste II2j6 |
| 200 | Mittelwertbildung, vorzeichengerecht |
| 200a | Eingabe: Liste II2j5 |
| 200b | Ausgabe: Mittelwert MWII2j5 |
| 210 | Mittelwertberechnung, vorzeichengerecht |
| 210a | Eingabe: Liste II2j6 |
| 210b | Ausgabe: Mittelwert MWII2j6 |
| 220 | Quotientenberechnung |
| 220a | Eingabe: Mittelwerte MWII2j5 und MWII2j6 |
| 220b | Ausgabe: Quotient Mj |
| 220c | Ausgabe: Liste III2 |
| 230 | Berechnung $\alpha j$ |
| 230a | Eingabe: Mittelwert Mj |
| 230b | Ausgabe: Winkel $\alpha j$ |
| 240 | nächste Teilliste, j = j + 1 |
| 250 | Mittelwertberechnung |
| 250a | Eingabe: Liste III2 |
| 250b | Ausgabe: Gesamtmittelwert |
| 260 | verfeinerte Teillistenbildung, i = i + 1 |
| 270 | R-R-Intervalldaten einlesen |

| | |
|---|---|
| 270a | Eingabe: EKG-Daten |
| 270a | Ausgabe: Liste LI1 |
| 280 | Letztes Element streichen |
| 280a | Eingabe: Liste LI1 |
| 280b | Ausgabe: Liste LII1 |
| 290 | Erstes Element streichen |
| 290a | Eingabe: Liste LI1 |
| 290b | Ausgabe: Liste LIII1 |
| 300 | Differenzbildung |
| 300a | Eingabe: Listen LIII1, LII1 |
| 300b | Ausgabe: Liste LIV1 |
| 310 | letztes Element streichen |
| 310a | Eingabe: Liste LIV1 |
| 310b | Ausgabe: Liste LV1 |
| 320 | erstes Element streichen |
| 320a | Eingabe: Liste LIV1 |
| 320b | Ausgabe: Liste LVI1 |
| 330 | Punktwolken-Darstellung |
| 330a | Eingabe: Listen LV1, LVI1 |
| 330b | Ausgabe: Punktwolkendiagramm |
| 340 | Mittelwertberechnung, vorzeichengerecht |
| 340a | Eingabe: Liste LV1 |
| 340b | Ausgabe: Mittelwert MWLV1 |
| 350 | Mittelwertberechnung, vorzeichengerecht |
| 350a | Eingabe: Liste LVI1 |
| 350b | Ausgabe: Mittelwert MWLVI1 |
| 360 | Quotientenbildung |
| 360a | Eingaben: Mittelwerte MWLV1 und MWLVI1 |
| 370 | Teilung in Teillisten |
| 370a | Eingabe: Liste LI1 |
| 370b | Ausgabe: Teillisten LI2, LI3,... |
| 380 | Entscheidung: Teilliste mit 4 Elementen links? |
| 390 | Mttelwertberechnung der Teillisten nach Schema in Fig. 1 |
| 390a | Eingabe: Teillisten LI2, LI3,... |
| 400 | R-Intervalldaten einlesen |
| 400a | Eingabe: EKG-Daten |
| 400b | Ausgabe: Liste RI1 |
| 410 | Letztes Element streichen |
| 410a | Eingabe: Liste RI1 |
| 410b | Ausgabe: Liste RII1 |
| 420 | Erstes Element streichen |
| 420a | Eingabe: Liste RI1 |
| 420b | Ausgabe: Liste RIII1 |
| 430 | Differenzbildung |
| 430a | Eingabe: Listen RIII1, RII1 |
| 430b | Ausgabe: Liste RIV1 |
| 440 | letztes Element streichen |
| 440a | Eingabe: Liste RIV1 |
| 440b | Ausgabe: Liste RV1 |
| 450 | erstes Element streichen |
| 450a | Eingabe: Liste RIV1 |
| 450b | Ausgabe: Liste RVI1 |
| 460 | Punktwolken-Darstellung |
| 460a | Eingabe: Listen RV1, RVI1 |
| 460b | Ausgabe: Punktwolkendiagramm |
| 470 | Mittelwertberechnung, vorzeichengerecht |
| 470a | Eingabe: Liste RV1 |
| 470b | Ausgabe: Mittelwert MWRV1 |

| | |
|---|---|
| 480 | Mittelwertberechnung, vorzeichengerecht |
| 480a | Eingabe: Liste RVI1 |
| 480b | Ausgabe: Mittelwert MWRVI1 |
| 490 | Quotientenbildung |
| 490a | Eingaben: Mittelwerte MWRV1 und MWRVI1 |
| 500 | Teilung in Teillisten |
| 500a | Eingabe: Liste RI1 |
| 500b | Ausgabe: Teillisten RI2, RI3,... |
| 510 | Etscheidung: Teilliste mit 4 Elementen links? |
| 520 | Mittelwertberechnung der Teillisten nach Schema in Fig. 1 |
| 520a | Eingabe: Teillisten RI2, RI3,... |
| 600 | Punktwolke, dicht regulär |
| 601 | Ordinate |
| 602 | Abszisse |
| 603 | Datenpunkt |
| 610 | Punktwolke verteilt, irregulär |
| 620 | erste Attraktorkurve mit erstem Krümmungsradius |
| 630 | zweite Attraktorkurve mit zweitem Krümmungsradius |
| 640 | Datenpunkt auf relativem Rhythmogrammaximum |
| 650 | Sattelpunkt |
| 650a | Lücke in extrahiertem Rhythmogramm |
| 651 | Datenpunkt auf relativen Rhythmogramminimum |
| 660 | relatives Maximum in extrahiertem Rhythmogramm |
| 670 | EKG-Daten einlesen |
| 670a | Eingabe: EKG-Daten |
| 680 | Bestimmen der relativen Maxima |
| 690 | Entscheidung: Maximum im Zählintervall? |
| 700 | ja: Zählintervall mit Zahlenwert 1 |
| 710 | nein: Zählintervall mit Zahlenwert 0 |
| 720 | Erstellen einer 0-1-Zahlenreihe |
| 730 | Mittelwertbildung über 0-1-Zahlenreihe |
| 740 | Ausgabe: Mittelwert, diagnostische Größe |

## Patentansprüche

**1.** Auswerteverfahren für Elektrokardiogrammdaten zur Aussage über einen funktionellen Gesamtzustand eines Organismus, zur Krebsfrüherkennung und/oder zur Anwendung in der Herz-Kreislauf-Diagnostik,
**dadurch gekennzeichnet, dass**
aus aufgezeichneten Daten eines Elektrokardiogramms (EKG) über eine Korrelationsanalyse eines zeitlich quasiperiodisch aufeinanderfolgenden Verlaufsmusters des EKG ein die Regelmäßigkeit dieses Verlaufsmusters beschreibender, auch diagnostisch aussagekräftiger, numerisch eindeutiger Wert gewonnen wird.

**2.** Auswerteverfahren nach Anspruch 1,
**dadurch gekennzeichnet, dass**
als Gegenstand der Korrelationsanalyse das quasiperiodische Verlaufsmuster einer zeitlichen Aufeinanderfolge systolischer R-Peaks des EKG analysiert wird.

**3.** Auswerteverfahren nach einem der vorhergehenden Ansprüche,
**dadurch gekennzeichnet, dass**
die Korrelationsanalyse folgende Schritte umfasst:

- Einlesen zeitlicher Daten aus dem EKG als Zeitintervalle $\tau_i$ zwischen aufeinanderfolgenden systolischen R-Peaks,
- Berechnung von Zeitintervalldifferenzen, durch mindestens eine Reihe erster Differenzbildungen $\tau_N-\tau_i$ und/oder eine Reihe zweiter Differenzbildungen $\tau_{N+n}-\tau_i$, wobei N,n und i beliebige ganze Zahlen sind,
- Erstellen eines mindestens zweidimensionalen Korrelationsdiagramms der Reihe der ersten Differenzbildung als Ordinatengröße über der Reihe der zweiten Differenzbildung als Abszissengröße,

- Errechnen mindestens eines ersten Mittelwertes beliebiger Art aus der Reihe der ersten Differenzbildungen und eines zweiten Mittelwertes beliebiger Art aus der Reihe der zweiten Differenzbildungen,
- Errechnen mindestens eines Quotienten aus dem ersten und dem zweiten errechneten Mittelwert,
- Ableiten eines diagnostisch aussagekräftigen Wertes $\alpha$ aus dem errechneten Quotienten der Mittelwerte.

**4.** Auswerteverfahren nach Anspruch 3,
**dadurch gekennzeichnet, dass**
die Korrelationsanalyse über einen vorgegebenen Zeitraum wiederholt zu Vergleichszwecken diagnostischer Befunde erfolgt, wobei frühere diagnostische Werte $\alpha$ mit späteren verglichen werden.

**5.** Auswerteverfahren für Elektrokardiogrammdaten zur Aussage über einen funktionellen Zustand des menschlichen Organismus, zur Krebsfrüherkennung und/oder zur Anwendung in der Herz-Kreislauf-Diagnostik,
**dadurch gekennzeichnet, dass**
aus Daten eines Elektrokardiogramms mindestens abschnittsweise eine zeitliche Verlaufsform der EKG-Kurve mittels eines iterativen Attraktormodells eines Parametersatzes mindestens einer Modellfunktion angepaßt wird, wobei nach der iterativ errechnete Parametersatz der Modellfunktion in einem Attraktordiagramm abgebildet wird.

**6.** Auswerteverfahren nach Anspruch 5,
**dadurch gekennzeichnet, dass**
als Modellfunktion mindestens ein Paar von Polynomen n-ten Grades (n ganzzahlig, beliebig) bezüglich der EKG-Zeitachsenvariable t verwendet werden, deren iterativ zu bestimmende Parametersätze miteinander gekoppelt sind.

**7.** Auswerteverfahren nach einem der Ansprüche 5 und 6,
**dadurch gekennzeichnet, dass**
das Attraktordiagramm des Parametersatzes stückweise eine Kurve ergibt, wobei mindestens ein Krümmungsradius an mindestens einem Kurvenstück als diagnostische Entscheidungsgröße bestimmt wird.

**8.** Auswerteverfahren nach einem der Ansprüche 5 bis 7,
**dadurch gekennzeichnet, dass**
der zeitliche Kurvenverlauf des EKG an den Q- und S-Peaks des Elektrokardiogramms analysiert wird, wobei mindestens eine Standardabweichung eines Mittelwertes der zeitlichen Abstände zwischen den R-Peaks des EKG als ein Startwert in den iterativ zu bestimmenden Parametersatz eingeht.

**9.** Auswerteverfahren für Elektrokardiogrammdaten zur Aussage über einen funktionellen Zustand des menschlichen Organismus, zur Krebsfrüherkennung und/oder zur Anwendung in der Herz-Kreislauf-Diagnostik
**dadurch gekennzeichnet, dass**
aus einer stetigen Zeitreihe von Intervallen quasiperiodischer EKG-Strukturen auftretende relative Maxima und/ oder Minima aus der EKG-Verlaufsform eines Rhythmogramms ausgewertet werden, wobei aus dem Rhythmogramm eine auf die Zählintervalle des der quasiperiodischen EKG-Strukturen bezogene Liste numerischer Daten erzeugt wird, die ein Auftreten eines relativen Maximums und/oder Minimums in jedem Zählintervall beschreiben, wobei aus dieser Liste ein numerischer diagnostisch aussagekräftiger Wert ermittelt wird.

**10.** Auswerteverfahren nach Anspruch 9,
**dadurch gekennzeichnet, dass**
als quasiperiodische EKG-Struktur die relativen Maxima des Rhythmogramms der stetigen Zählreihe der R-R-Intervalle ausgewertet werden, wobei eine zeitliche Intervalllänge der R-R-Abstände in Abhängigkeit von einer Zahl der R-R-Abstände als Rhythmogramm ausgewertet wird.

**11.** Auswerteverfahren nach Anspruch 10,
**dadurch gekennzeichnet, dass**
die relativen Maxima des Rhythmogramms auf einen Zahlenwert normiert werden.

**12.** Auswerteverfahren nach einem der Ansprüche 9 bis 11,
**dadurch gekennzeichnet, dass**
bei einem im normierten Rhythmogramm in einem Zählintervall festgestellten Maximum diesem Zählintervall ein erster numerischer Wert beigelegt wird und bei einem nicht vorliegenden Maximum in einem Zählintervall diesem Zählintervall ein zweiter numerischer Wert beigelegt wird, wobei eine Zahlenreihe aus wechselnden numerischen Werten erzeugt wird.

13. Auswerteverfahren nach einem der Ansprüche 9 und 12,
   **dadurch gekennzeichnet, dass**
   aus der Zahlenreihe ein Mittelwert gebildet wird, der als diagnostische Größe dient.

Stand der Technik

Fig. 1.

Fig. 2a

Fig. 2b

Fig. 3a

Fig. 3b

19

Fig. 3c

Fig. 4a

Fig. 4b

Fig. 5a

**460a**

Liste RV1
Liste RVI1

**460**

Darstellung

RV1 = y
RVI1 = x

**460·b**

Punktwolkendiagramm

**470a**

Liste RV1

**470**

Mittelwertberechnung

MWRV1

**470·b**

MWRV1

**480a**

Liste RVI1

**480**

Mittelwertberechnung

MRVI1

**480·b**

MWRVI1

**490a**

MWRV1
MWRVI1

**490**

Quotient
M13 =
- MWRV1/
(MWRVI1+e)

**490·b**

M13

**500a**

Liste RI1

**500**

Teilung in
Teillisten

**500·b**

Teillisten
RI2,RI3,...

**510**

Teilliste
mit 4 Elementen
rechts?

n

j

**520a**

Teillisten
RI2,RI3,...

**520**

Mittelwertberechnung

ENDE

Fig. 5b

24

Fig. 6a

F 30
(III)

Fig. 6b

Y - Achse

2.5

2

1.5          Ra=3.2793

1

0.5

2.91   2.92   2.93   2.94   2.95

620

X - Achse

Attraktor - Modell

*Fig. 7a*

Y

2.5

2

1.5

1          Ra= -0.264861

630

0.5

3.15   3.2   3.25   3.3   3.35

X - Achse

Attraktormodell

*Fig. 7b*

Fig. 8a

Fig. 8b

Fig. 9a

Fig. 9b

28

Fig. 10

Europäisches
Patentamt

**EUROPÄISCHER RECHERCHENBERICHT**

Nummer der Anmeldung

EP 03 00 0093

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | PENG C.-K. ET AL: "Long-Range Anticorrelations and Non-Gaussian Behaviour of the Heartbeat" PHYSICAL REVIEW LETTERS, Bd. 70, Nr. 9, 1. März 1993 (1993-03-01), Seiten 1343-1346, XP002240020 | 1,2 | A61B5/0452 G06F17/00 |
| A | * Seite 1343, linke Spalte, Absatz 2 - rechte Spalte, Absatz 3 * --- | 3,4 | |
| X | US 5 755 671 A (ALBRECHT PAUL ET AL) 26. Mai 1998 (1998-05-26) | 1,2 | |
| A | * Ansprüche 34,36,49 * * Spalte 8, Zeile 44-59 * * Spalte 9, Zeile 30-51 * --- | 3,4 | |
| A | EP 0 621 540 A (BABLOYANTZ AGNESSA) 26. Oktober 1994 (1994-10-26) * Seite 2, Zeile 52 - Seite 4, Zeile 23 * * Abbildung 3 * --- | 1-4 | |
| A | US 5 622 178 A (GILHAM JEFFREY J) 22. April 1997 (1997-04-22) * Spalte 2, Zeile 10-27 * * Spalte 8, Zeile 26-37 * * Abbildung 6 * --- | 1-4 | RECHERCHIERTE SACHGEBIETE (Int.Cl.7) A61B G06F |
| A | US 4 934 374 A (DEPAOLA ROBERT ET AL) 19. Juni 1990 (1990-06-19) * Spalte 2, Zeile 8 - Spalte 3, Zeile 22 * ----- | 1-4 | |

~~Der vorliegende Recherchenbericht wurde für alle Patentansprüche erstellt~~

| Recherchenort | Abschlußdatum der Recherche | Prüfer |
|---|---|---|
| MÜNCHEN | 5. Mai 2003 | Völlinger, M |

**Europäisches
Patentamt**

## GEBÜHRENPFLICHTIGE PATENTANSPRÜCHE

Die vorliegende europäische Patentanmeldung enthielt bei ihrer Einreichung mehr als zehn Patentansprüche.

☐ Nur ein Teil der Anspruchsgebühren wurde innerhalb der vorgeschriebenen Frist entrichtet. Der vorliegende europäische Recherchenbericht wurde für die ersten zehn sowie für jene Patentansprüche erstellt, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die ersten zehn Patentansprüche erstellt.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

1-4

| | | |
|---|---|---|
| **Europäisches**<br>**Patentamt** | **MANGELNDE EINHEITLICHKEIT**<br>**DER ERFINDUNG**<br>**ERGÄNZUNGSBLATT B** | Nummer der Anmeldung<br>EP 03 00 0093 |

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-4

   Auswerteverfahren für Elektrokardiogrammdaten mittels Korrelationsanalyse

2. Ansprüche: 5-8

   Auswerteverfahren für Elektrokardiogrammdaten mittels Anpassung einer Modellfunktion

3. Ansprüche: 9-13

   Auswerteverfahren für Elektrokardiogrammdaten mittels Analyse der relativen Maxima und/oder Minima von Intervallen quasiperiodischer EKG-Strukturen

ANHANG ZUM EUROPÄISCHEN RECHERCHENBERICHT
ÜBER DIE EUROPÄISCHE PATENTANMELDUNG NR.

EP 03 00 0093

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-05-2003

| Im Recherchenbericht angeführtes Patentdokument | | Datum der Veröffentlichung | Mitglied(er) der Patentfamilie | | Datum der Veröffentlichung |
|---|---|---|---|---|---|
| US 5755671 | A | 26-05-1998 | EP<br>WO | 0801538 A<br>9712546 A | 22-10-1997<br>10-04-1997 |
| EP 0621540 | A | 26-10-1994 | KEINE | | |
| US 5622178 | A | 22-04-1997 | KEINE | | |
| US 4934374 | A | 19-06-1990 | KEINE | | |

EPO FORM P0461

Für nähere Einzelheiten zu diesem Anhang : siehe Amtsblatt des Europäischen Patentamts, Nr.12/82